(19) Europäisches Patentamt
European Patent Office
Office européen des brevets

(11) Publication number: **0 140 646**
**B1**

(12) **EUROPEAN PATENT SPECIFICATION**

(45) Date of publication of patent specification: **12.08.87**

(21) Application number: **84307130.9**

(22) Date of filing: **17.10.84**

(51) Int. Cl.[4]: **C 07 C 103/19,**
C 07 C 102/00, C 07 C 119/06,
C 07 D 221/20

(54) **Process for producing 9-carbamoyl fluorene derivatives.**

(30) Priority: **19.10.83 US 543200**
**19.10.83 US 543648**

(43) Date of publication of application:
**08.05.85 Bulletin 85/19**

(45) Publication of the grant of the patent:
**12.08.87 Bulletin 87/33**

(84) Designated Contracting States:
**AT BE CH DE FR GB IT LI LU NL SE**

(56) References cited:
**FR-A- 38 676**
**FR-A-2 457 278**

**CHEMICAL ABSTRACTS, vol. 36, no. 18, September 20, 1942, Columbus, Ohio, USA O. EISLEB "New synthesis with sodium amide" columns 5465-5468**

(73) Proprietor: **ELI LILLY AND COMPANY**
**307, East McCarty Street**
**Indianapolis Indiana 46285 (US)**

(72) Inventor: **Lacefield, William Bryant**
**636 Boulder Road**
**Indianapolis, IN 46217 (US)**
Inventor: **Lindstrom, Terry Donald**
**1335 West 79th Street**
**Indianapolis, IN 46260 (US)**
Inventor: **Johnson, Derek**
**15 Carlingford Road Walton**
**Warrington Cheshire (GB)**
Inventor: **Spreadbury, Alan Charles**
**32 Norton Avenue Penketh**
**Warrington Cheshire (GB)**

(74) Representative: **Hudson, Christopher Mark et al**
**Erl Wood Manor**
**Windlesham Surrey GU20 6PH (GB)**

**EP 0 140 646 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

A group of 9-disubstituted fluorenes recently has been discovered to have potent antiarrhythmic activity; see U.S. Patent Nos. 4,197,313 and 4,382,093. The 9-carbamoyl-9-aminoalkylfluorenes are among the most active compounds, and one compound within this group, namely 9-(3-isopropylaminopropyl)-9-carbamoylfluorene hydrochloride, is now known generically as indecainide. The reported synthesis of the 9-carbamoyl-9-aminoalkylfluorenes have included acid hydrolysis of a 9-cyano-9-aminoalkylfluorene, and more recently reductive alkylation of a 9-carbamoyl-9-cyanoalkylfluorene, see U.S. Patent No. 4,282,170.

The invention provides a process for producing a compound of the formula

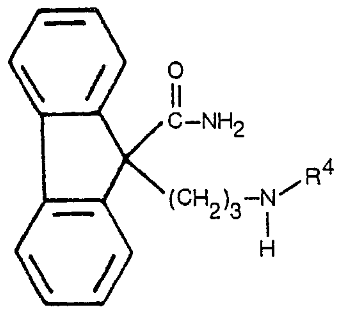

(I)

wherein

$R^4$ is $C_1$—$C_6$ alkyl; or a salt thereof; which comprises reacting a compound of the formula

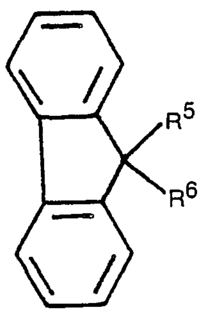

(II)

wherein

either $R^5$ is —$CONH_2$ and $R^6$ is —$(CH_2)_2CHO$; or $R^5$ and $R^6$, together, form the ring

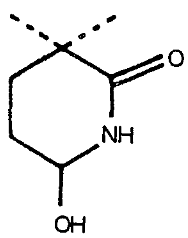

with an amine of the formula $R^4NH_2$ and a reducing agent; or isolating an intermediate of the following formula from reaction of the compound of formula (II) with amine:

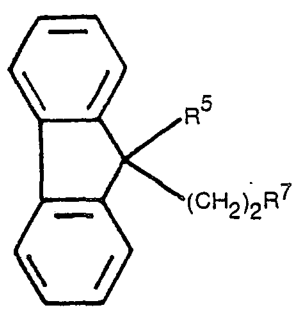

(III)

where $R^7$ is —$CH(OH)NHR^4$ or —$CH{=}NR^4$ and reducing the said intermediate.

The compounds of formula (II) are of two types;

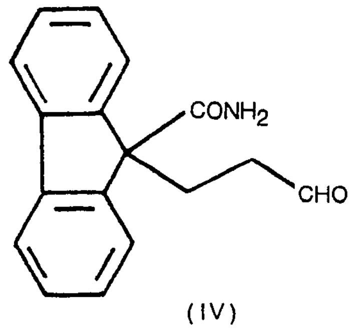
(IV)

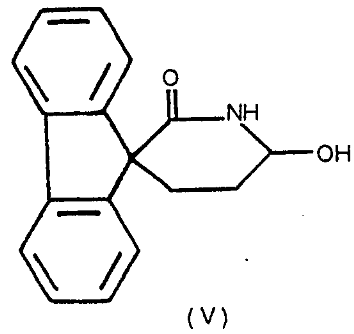
(V)

Such compounds are in fact interchangeable since reaction of base on compound (V) readily yields the aldehyde of formula (IV). In most systems an equilibrium between the two forms exists and the reactive species in the above process of the invention is probably always of formula (IV). Notwithstanding this both species can be employed in the reaction. Amine ($R^4NH_2$) and reducing agent can be added simultaneously or the reducing agent added after reaction with amine.

The compounds of formula (III) are products of the reaction of amine with the compound of formula (II) and can be isolated and subsequently reacted with reducing agent. Often, however, it is preferable to avoid the extra step of isolating the intermediate product and to carry out the reaction *in situ* as described above.

The compounds of formula (II) and (III) are novel and are provided in one aspect of the invention.

The compounds of formulae (II) can be prepared by a process which comprises reacting a compound of the formula

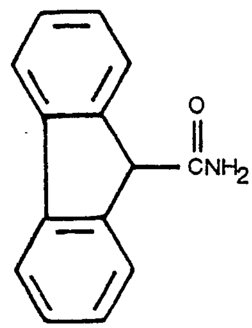

with acrolein ($CH_2$=CHCHO) under basic conditions.

The compounds of formula (I) as defined above can be prepared by a process which comprises

(a) reacting a compound of the formula

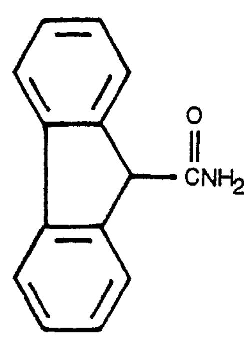

with acrolein under basic conditions to provide a compound of formula;

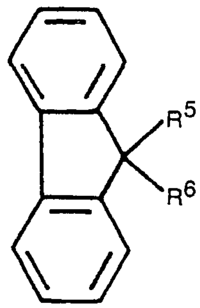

(II)

wherein $R^5$ and $R^6$ are as defined above, and

(b) reacting the compound of formula (II) with an amine of the formula $R^4NH_2$ and a reducing agent.

In the above formulae $R^4$ is $C_1$—$C_6$ alkyl. The term "$C_1$—$C_6$ alkyl" carries its art-recognized meaning and includes methyl, *iso*propyl, *tert.*-butyl and 1,1-dimethylbutyl. A particularly preferred $R^4$ alkyl group is *iso*propyl.

The invention includes a process for producing a compound of formula (II) which comprises reacting a compound of the formula

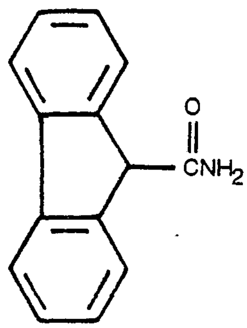

with acrolein under basic conditions.

The reaction of amine with the compound of formula (II) above, is one of amination and, in the case where an intermediate is not isolated, the reaction is one of reductive amination. It is preferably carried out with approximately equimolar quantities of the reactants or with an excess of the amine, at a pH within the range of 6 to 10, such as for example 8 to 9, and at a temperature of from −20°C to 100°C, such as for example from 25°C to 80°C. It is preferred to perform the reaction under basic conditions which may be ensured by the use of a molar excess of the amine reactant. The subsequent reduction in the case where an intermediate is isolated is carried out under similar conditions.

Suitable reducing agents or use in the above process can be catalytic or chemical. Examples of the former kind include hydrogenation with palladium and charcoal, hydrogen and Adams catalyst ($PtO_2$), and platinum on charcoal. Such catalytic methods of hydrogenation are discussed for instance in "Practical Catalytic Hydrogenation" by M. Friefelder, Wiley Interscience, 1971, and in "Catalytic Hydrogenation in Organic Synthesis" by P.N. Rylander, Academic Press, 1979. Chemical reagents which may preferably be used include, for example, sodium cyanoborohydride, lithium cyanoborohydride, tetrabutylammonium cyanoborohydride. The first named reagent, which is the most preferred for use in the process of the invention, is reviewed, for example, in the paper by C. F. Lane, Aldrichimica Acta 1975, *8*, 3. Reducing agents with too strong an effect should be avoided.

The reaction is generally carried out in an inert organic solvent and we have found that acetonitrile, dichloromethane and ethyl acetate are particularly useful for this purpose but other solvents can be employed such as for example tetrahydrofuran and aromatic and halogenated hydrocarbons, or if desired the amine reactant can be employed in sufficient quantity to serve both as reactant and solvent. The product can be extracted by routine procedures.

As noted above compounds of formula (II) can be prepared by a novel process which comprises reaction with acrolein according to the following scheme

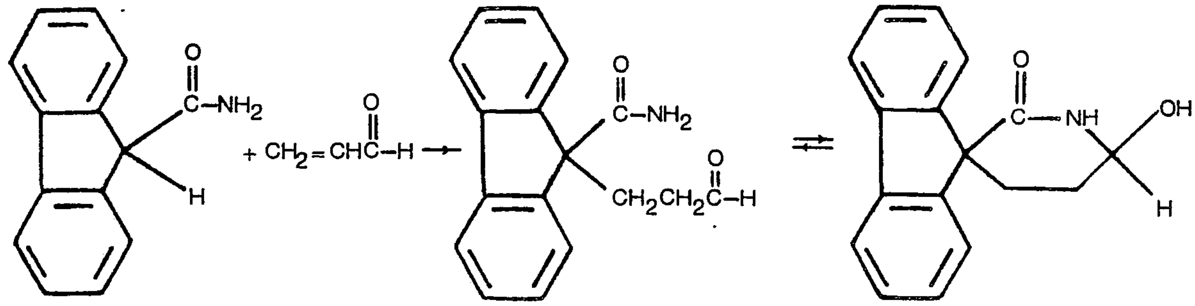

The reaction is carried out by combining the 9-carbamoylfluorene with acrolein in approximately equimolar quantities in the presence of a base. The particular base employed and the quantity are not critical, and an equimolar quantity or excess generally is used. Typical bases that can be employed include organic bases such as benzyltrimethylammonium hydroxide (Triton B), triethylamine, as well as alkali metal hydrides such as sodium hydride. The reaction is preferably conducted in an organic solvent, and typical solvents include ethers such as diethyl ether and tetrahydrofuran, and aromatics such as benzene and toluene. The reaction typically is carried out at a temperature of about 0 to 100°C, and at such temperature is usually complete within about on to about eight hours. The product of the reaction, a tautomeric mixture of an aldehyde and a cyclic carbinol, is readily isolated by removing the reaction solvent or when a water-miscible solvent has been employed, by dilution of the reaction mixture with water. The product can be crystallized from common solvents such as for example ethyl acetate in ether, when it is obtained predominantly in the carbinol form (formula V)) which as such can be readily isolated.

The following detailed Examples further illustrate this invention.

## Example 1

6′-Hydroxyspiro(9H-fluorene-9,3′-piperidine)-2′-one

A solution of 10.0 g (48 mM) of 9-carbamoylfluorene in 100 ml of tetrahydrofuran was heated to 45°C and stirred under a nitrogen blanket with 2 ml of N-benzyltrimethyl ammonium hydroxide (Triton B) were added in one portion. The reaction mixture was stirred for fifteen minutes at 45°C, and then 2.8 g (50 mM) of acrolein were added dropwise over ten minutes. The reaction mixture was heated at reflux for three hours following the addition. The reaction mixture was cooled to room temperature and concentrated to an oil by evaporation of the solvent under reduced pressure. The oil was dissolved in 100 ml of ethyl acetate and washed with water. The organic layer was dried and the solvent was removed by evaporation to provide a solid. The solid was crystallized from ethyl acetate and petroleum ether to give 4.0 g of solid product. The product was purified further by high pressure liquid chromatogrpahy over silica gel, eluting with chloroform containing 5% (v/v) methanol. The appropriate fractions were combined and concentrated to dryness to afford 500 mg of 6′-hydroxyspiro-(9H-fluorene-9,3′-piperidine)-2′-one melting at 210—212°C.

Analysis calculated for $C_{17}H_{15}NO_2$ (after drying at 120°C):
Theory: C, 76.96; H, 5.70; N, 5.28; O, 12.06
Found: C, 76.66; H, 5.99; N, 5.21; O, 12.27.

Mass Spec. $M^+$ Theory 265, Found 265.
NMR ($DMSOd_6$): δ 1.6—2.5 (m, 4H); 5.32 (broad 1H): 6.12 (d, 1H); 7.3—8.1 (m, 8H); 8.32 (d, 1H).
IR (KBr): 1642 $cm^{-1}$ (amide).

## Example 2

6′-Hydroxyspiro(9H-fluoroene-9,3′-piperidine)-2′-one

Following the general procedure of Example 1, 20.9 g (0.1 mole) of 9-carbamoylfluorene were added to 300 ml of tetrahyrofuran. The solution was heated to 50°C and stirred while 6 ml of benzyltrimethylammonium hydroxide were added in one portion. After stirring the reaction mixture at 50°C for thirty minutes, 6.2 g (0.11 mole) of acrolein were added and the mixture was then heated at reflux for four hours. The reaction mixture was cooled to 25°C and the solvent was removed by evaporation under reduced pressure to provide 29.9 g of 6′-hydroxyspiro(9H-fluorene-9,3′-piperidine)-2′-one and its tautomeric isomer 9-carbamoyl-9-(3-oxo-propyl)-fluorene, m.p. 199—201°C.

Analysis calculated for $C_{16}H_{15}NO_2$ (after drying at 120°C for one minute).
Theory: C, 76.96; H, 5.70; N, 5.28
Found: C, 76.71; H, 5.88; N, 5.23.

Mass Spec. $M^+$ Theory 265; Found 265.
NMR ($CDCl_3$ + $DMSOd_6$): δ 1.7—2.6 (m, 4H) 5.3 (broad s, 1H); 5.9 (d, 1H); 7.2—7.9 (m, 9H).
IR (KBr): 1693 $cm^{-1}$ (amide).

## Example 3

9-Carbamoyl-9-(3-isopropylaminopropyl)fluorene hydrochloride

A solution of isopropylamine (45.0 ml) in acetonitrile (250 ml) was cooled to 10°C and hdyrogen chloride gas introduced into the solution keeping the temperature below 15°C to pH 8.5 ± 0.3. The resultant slurry was allowed to warm to room temperature and sodium cyanoborohydride (12.0 g) added in one portion and the pH readjusted to 8.5 if necessary. 6′-Hydroxyspiro(9H-fluoroene-9,3′-piperidine)-2′-one (30.0 g) was then added and the reaction mixture heated to reflux for 2 hours. The mixture was allowed to cool to room temperature and dilute hdyrochloric acid added to pH 1—2 to destroy any excess cyanoborohydride. The acetonitrile was removed under reduced pressure and the residue basified with sodium hydroxide solution (pH 11) and extracted with dichloromethane (3 × 100 ml) and the combined orgnaic layers washed with sodium hydroxide solution, dried with anhydrous sodium sulphate and evaporated to give the crystalline free amine.

This amine was dissolved in acetone (120 ml) and cooled to 15°C. Dry hydrogen chloride gas was passed through the solution until a pH of 7 was obtained. The resultant mixture was cooled slowly to −30°C, and the product isolated by filtration, washed with cold acetone, and dried *in vacuo* at 40°C overnight to give the title compound, yield 25.4 g, 65%.

NMR ($DMSOd_6$): δ 1.08 (s, 3H); 1.19 (s, 3H); 2.2—2.9 (m, 6H); 6.28 (s, 1H); 7.0 (s, 1H); 7.3—8.1 (m, 9H); 8.8 (broad s, 2H).

Example 4

9-Carbamoyl-9-(3-isopropylaminopropyl)fluorene

6'-Hydroxyspiro-(9H-fluorene-9,3'-piperidine)-2'-one (2.7 g) was dissolved in isopropylamine (350 ml) and 10% palladium on charcoal (0.3 g) was added and the vessel pressurized to $6.2 \times 10^5$ Pa (90 p.s.i.) with hydrogen. The reaction mixture was then heated at 40°C for 16 hours and then allowed to cool to room temperature. The catalyst was removed by filtration and the isopropylamine evaporated under reduced pressure. The resulting residue was dissolved in dichloromethane (20 ml) washed with water (2 × 50 ml), dried with anhydrous sodium sulphate and evaporated to dryness under reduced pressure. The residue was triturated with hexane to give the title compound as white crystals, yield 2.0 g, 64%, the identity of which was confirmed by thin layer chromatographic comparison with an authentic sample (silica gel, ethylacetate:methanol 80:20 v/v).

Example 5

9-Carbamoyl-9-(3-isopropyliminopropyl)fluorene

A mixture of 5.9 g (0.1 mole) of isopropylamine and 2.5 g (0.009 mole) of the tautomeric mixture from Example 2 was stirred at 25°C under a nitrogen blanket for sixteen hours. Excess isopropylamine was removed by evaporation under reduced pressure to provide, following crystallization from ethyl acetate and petroleum ether, 0.7 g of a solid identified as 9-carbamoyl-9-(3-isopropyliminopropyl)fluorene, m.p. 149—155°C.

M+ theory 306; found 306.

NMR ($CDCl_3$): signals at δ 1.0 (two s, 6H); 1.62 (M, 2H); 2.65 (M, 2H); 3.06 (M, 1H); 5.05 (Broad s, 2H, signal removed with $D_2O$ shake); 7.28—7.79 (M, 9H).

Example 6

9-Carbamoyl-9-(3-isopropylaminopropyl)fluorene hydrochloride

9-Carbamoyl-9-(3-isopropyliminopropyl)fluorene prepared as in Example 5, was dissolved in 200 ml of ethanol containing 3.0 g of 5% palladium on carbon. The reaction mixture was shaken in a hydrogen atmosphere at 25°C for six hours. The actual hdyrogen uptake was 8.94 kg (19.5 lbs); theoretical uptake was 8.94 kg (19.7 lbs). The reaction mixture was filtered to remove the hydrogenation catalyst and the filtrate was concentrated to dryness by evaporation under reduced pressure. The solid that was formed was dissolved in 100 ml of diethyl ether containing 100 ml of ethyl acetate. The product was extracted into 6N hydrochloric acid and the acid extracts were combined, cooled to 10°C and made alkaline to pH 10.0 by addition of 10% (w/v) aqueous sodium hydroxide. The alkaline solution was extracted several times with fresh diethyl ether, and the etheral extracts were combined, washed with water, dried, and then saturated with gaseous hdyrogen chloride. The precipitate which formed was collected by filtration and recrystallized from ethanol and diethyl ether to give 2.1 g of 9-carbamoyl-9-(3-isopropylaminopropyl)fluorene hydrochloride (indecainide hydrochloride), m.p. 206—207.5°C.

Analysis calculated for $C_{20}H_{25}ClN_2O$
Theory: C, 69.65; H, 7.31; H, 8.12
Found: C, 69.55; H, 7.18; N, 7.99.

IR (KBr): 1680, 1665 $cm^{-1}$.

NMR ($DMSOd_6$): δ 1.08 (s, 3H); 1.19 (s, 3H); 2.2—2.9 (m. 6H); 6.28 (s, 1H); 7.0 (s, 1H); 7.3—8.1 (m, 9H); 8.8 (broad s, 2H).

Titration (66% v/v N,N-dimethylformamide-water) pKa = 10.2.

**Claims**

1. A process for producing a compound of the formula

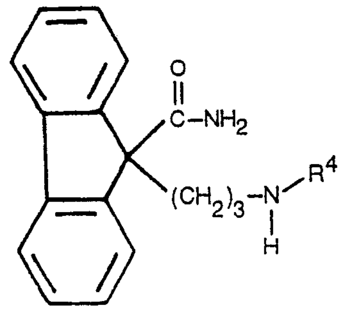

(I)

wherein

$R^4$ is $C_1$—$C_6$ alkyl; or a salt thereof; which comprises reacting a compound of the formula

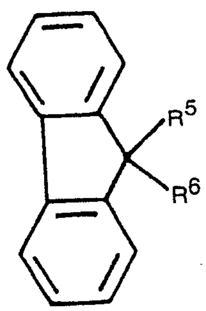

(II)

wherein

either $R^5$ is $—CONH_2$ and $R^6$ is $—(CH_2)_2CHO$; or $R^5$ and $R^6$, together, form the ring

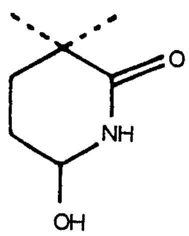

with an amine of the formula $R^4NH_2$ and a reducing agent; or isolating from reaction of the compound of formula (II) with amine, an intermediate of the formula:

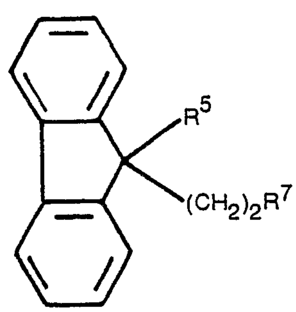

(III)

where $R^7$ is $—CH(OH)NHR^4$ or $—CH{=}NR^4$ and reducing the said intermediate.

2. A process according to claim 1 for preparing a compound of the formula (I) in which $R^4$ is isopropyl.

3. A process for producing a compound of formula (II)

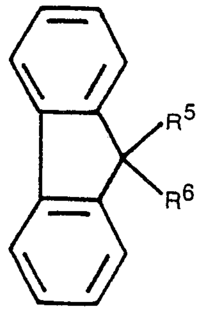

(II)

wherein

either $R^5$ is —$CONH_2$ and $R^6$ is —$(CH_2)_2CHO$; or $R^5$ and $R^6$, together, form the ring

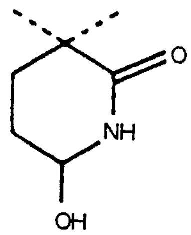

which comprises reacting a compound of the formula

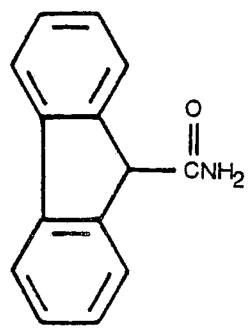

with acrolein undr basic conditions.

4. A compound of formula (III):

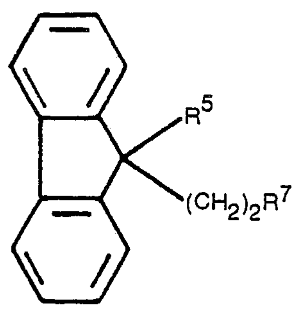

(III)

where $R^7$ is —CH(OH)$NHR^4$ or —CH=$NR^4$ where $R^4$ is $C_{1-6}$ alkyl.

5. A compound of formula (III) as claimed in claim 4, wherein $R^7$ is —CH=$NR^4$, where $R^4$ is isopropyl.

6. A compound of formula (II):

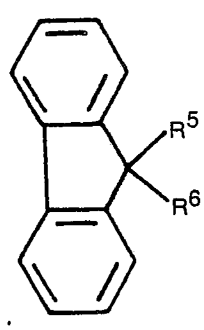

(II)

wherein either $R^5$ is $CONH_2$ and $R^6$ is $—(CH_2)_2CHO$; or $R^5$ and $R^6$, together, form the ring:

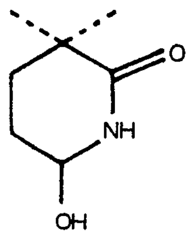

**Patentansprüche**

1. Verfahren zur Herstellung einer Verbindung der Formel

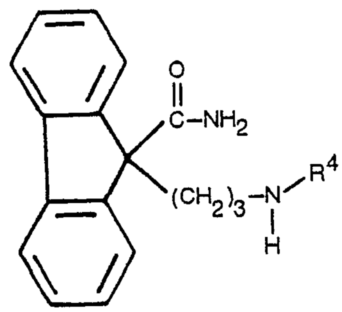

(I)

worin $R^4$ für $C_1—C_6$-Alkyl steht, oder eines Salzes hiervon, dadurch gekennzeichnet, daß man eine Verbindung der Formel

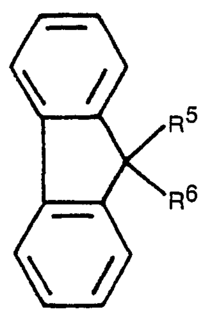

(II)

worin entweder $R^5$ für $—CONH_2$ steht und $R^6$ für $—(CH_2)_2CHO$ steht, oder $R^5$ und $R^6$ zusammen den Ring

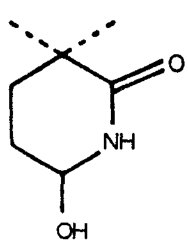

bilden, mit einem Amin der Formel $R^4NH_2$ und einem Reduktionsmittel umsetzt oder aus einer Umsetzung der Verbindung der Formel (II) mit einem Amin ein Zwischenprodukt der Formel

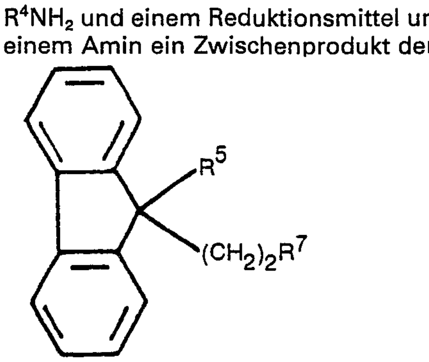

(III)

worin $R^7$ für $—CH(OH)NHR^4$ oder $—CH{=}NR^4$ steht, isoliert und dieses Zwischenprodukt reduziert.

2. Verfahren nach Anspruch 1, dadurch gekennzeichnet, daß man eine Verbindung der Formel (I) herstellt, worin $R^4$ Isopropyl ist.

3. Verfahren zur Herstellung einer Verbindung der Formel (II)

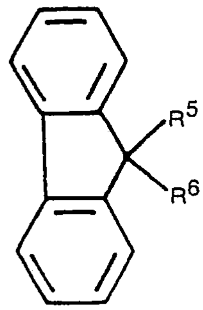

(II)

worin entweder $R^5$ für $—CONH_2$ steht und $R^6$ für $—(CH_2)_2CHO$ steht oder $R^5$ und $R^6$ zusammen den Ring

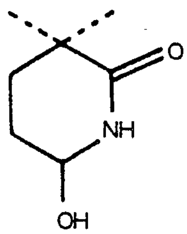

bilden, dadurch gekennzeichnet, daß man eine Verbindung der Formel

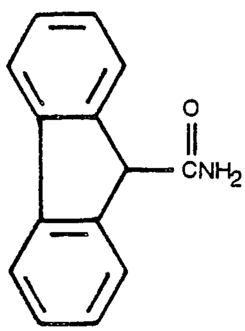

mit Acrolein unter basischen Bedingungen umsetzt.

4. Verbindung der Formel (III)

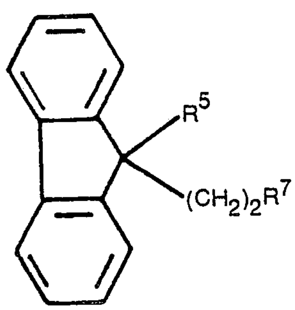

(III)

worin $R^7$ für $—CH(OH)NHR^4$ oder $—CH{=}NR^4$ steht, wobei $R^4$ für $C_1—C_6$-Alkyl steht.

5. Verbindung der Formel (III) gemäß Anspruch 4, dadurch gekennzeichnet, daß $R^7$ für $—CH{=}NR^4$ steht, wobei $R^4$ Isopropyl ist.

6. Verbindung der Formel (II)

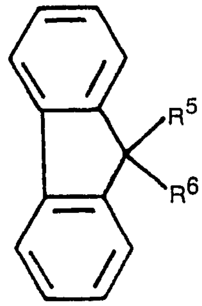

(II)

worin entweder $R^5$ für $CONH_2$ steht und $R^6$ für $-(CH_2)_2CHO$ steht oder $R^5$ und $R^6$ zusammen den Ring

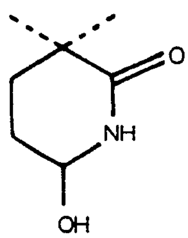

bilden.

**Revendications**

1. Procédé de préparation d'un composé de formule:

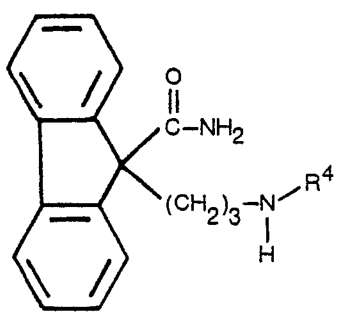

(I)

dans laquelle
$R^4$ représente un groupe alkyle en $C_1$—$C_6$; ou d'un de ses sels; caractérisé en ce qu'il consiste à faire réagir un composé de formule:

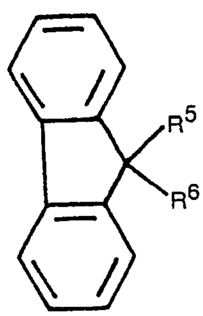

(II)

dans laquelle
$R^5$ représente $-CONH_2$ et $R^6$ représente $-(CH_2)_2CHO$;
ou $R^5$ et $R^6$, ensemble, forment le noyau

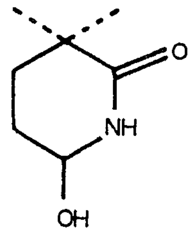

avec une amine de formula $R^4NH_2$ et un agent réducteur; ou isoler, de la réaction du composé de formule (II) avec une amine, un produit intermédiaire de formule:

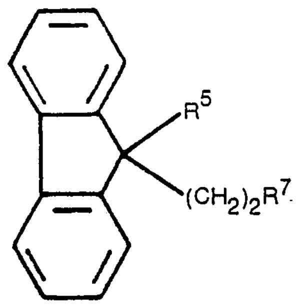

(III)

dans laquelle $R^7$ représente —CH(OH)NHR$^4$ ou —CH=NR$^4$ et réduire ce produit intermédiaire.

2. Procédé selon la revendication 1 pour préparer un composé de formule (I) dans laquelle $R^4$ est le groupe isopropyle.

3. Procédé de préparation d'un composé de formule (II):

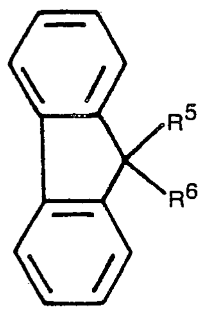

(II)

dans laquelle $R^5$ représente —$CONH_2$ et $R^6$ représente —$(CH_2)_2CHO$;
ou $R^5$ et $R^6$, ensemble, forment le noyau

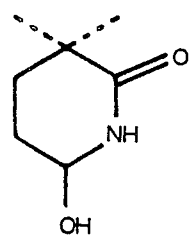

caractérisé en ce qu'il consiste à faire réagir un composé de formule:

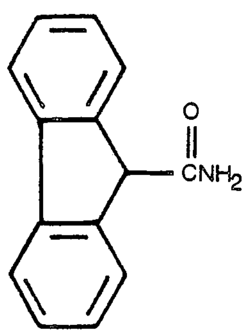

avec l'acroléine dans des conditions basiques.

4. Composé de formule (III):

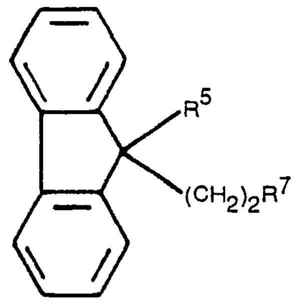

(III)

dans laquelle $R^7$ représente $-CH(OH)NHR^4$ ou $-CH=NR^4$ où $R^4$ représente un groupe alkyle en $C_1-C_6$.

5. Composé de formule (III) selon la revendication 4, dans laquelle $R^7$ représente $-CH=NR^4$ où $R^4$ est le groupe isopropyle.

6. Composé de formule (II):

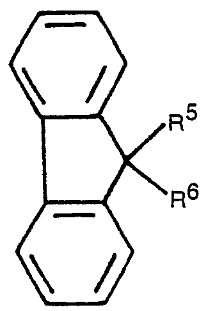

(II)

dans laquelle $R^5$ représente $CONH_2$ et $R^6$ représente $-(CH_2)_2CHO$; ou $R^5$ et $R^6$, ensemble, forment le noyau;